(19) Europäisches Patentamt

European Patent Office

Office européen des brevets

(11) Veröffentlichungsnummer: **0 502 916 B1**

(12) **EUROPÄISCHE PATENTSCHRIFT**

(45) Veröffentlichungstag der Patentschrift: **25.01.95**

(21) Anmeldenummer: **90917723.0**

(22) Anmeldetag: **19.11.90**

(86) Internationale Anmeldenummer:
**PCT/EP90/01975**

(87) Internationale Veröffentlichungsnummer:
**WO 91/08193 (13.06.91 91/13)**

(51) Int. Cl.⁶: **C07C 227/40**, C07C 231/24

(54) **VERFAHREN ZUR NACHBEHANDLUNG AMPHOTERER ODER ZWITTERIONISCHER TENSIDE.**

(30) Priorität: **28.11.89 DE 3939264**

(43) Veröffentlichungstag der Anmeldung:
**16.09.92 Patentblatt 92/38**

(45) Bekanntmachung des Hinweises auf die
Patenterteilung:
**25.01.95 Patentblatt 95/04**

(84) Benannte Vertragsstaaten:
**CH DE DK ES FR GB IT LI NL SE**

(56) Entgegenhaltungen:
**EP-A- 0 243 619**
**US-A- 3 928 447**

**CHEMICAL ABSTRACTS, Band 92, Nr. 11, 17.
März 1980, Columbus, Ohio, US; S. 545, Zusammenfassung Nr. 93884d; & SU-A-702 003**

(73) Patentinhaber: **Henkel Kommanditgesellschaft
auf Aktien**

**D-40191 Düsseldorf (DE)**

(72) Erfinder: **UPHUES, Günter
Robert-Koch-Str. 45
D-4019 Monheim (DE)**
Erfinder: **PLOOG, Uwe
Haydnweg 6
D-5657 Haan (DE)**
Erfinder: **BISCHOF, Klaudia
Selmerstr. 10
D-4712 Werne (DE)**
Erfinder: **KENAR, Kenan
Kamerathsfeldstr. 6
D-4000 Düsseldorf 13 (DE)**
Erfinder: **SLADEK, Pavel
Andreasstr. 55
D-5160 Düren 8 (DE)**

**Beschreibung**

Die Erfindung betrifft ein Verfahren zur Erniedrigung des Restgehaltes an freiem Alkylierungsmittel in wässrigen Lösungen amphoterer oder zwitterionischer Tenside durch Nachbehandlung der Lösungen mit Ammoniak, einer Aminosäure mit 2 bis 8 Kohlenstoffatomen oder einem Oligopeptid.

Amphotere und zwitterionische Tenside sind durch eine molekulare Struktur gekennzeichnet, die zwei funktionelle Gruppen unterschiedlicher Polarität, in der Regel eine kationische Ammoniumgruppe und eine anionische Carboxylat- oder Sulfonatfunktion enthalten. Tenside mit amphoterer oder zwitterionischer Struktur verbinden ein ausgezeichnetes Reinigungsvermögen mit guter dermatologischer Verträglichkeit. Da sie ferner in wässiger Lösung einen dichten, stabilen Schaum bilden, der auch in Gegenwart von Seife nicht zusammenbricht, stellen diese Klassen von Tensiden wertvolle Rohstoffe für die Herstellung von Körperreinigungsmitteln, insbesondere Haarshampoos und Duschbädern dar.

Zur Synthese amphoterer oder zwitterionischer Tenside geht man von primären, sekundären oder tertiären Aminoverbindungen aus, die mit einem geeigneten Alkylierungsmittel umgesetzt werden [J.Falbe (ed.), "Surfactants in consumer products", Springer Verlag, Berlin-Heidelberg, 1987, S.114f].

So erhält man z. B. aus der Quaternierung von tertiären Aminen [Seifen-Öle- Fette-Wachse, **108**, 373 (1982)] oder Fettsäureamidoaminen [Parf.Kosm.Arom. **75**, 67 (1968)] mit Halogencarbonsäuren, Alkylbetaine bzw. Alkylamidobetaine mit amphoterer Struktur. Von besonderer technischer Bedeutung sind zwitterionische Tenside auf Basis von Alkylimidazolinen, die mit Halogencarbonsäuren [DE 36 39 752 A1] oder Acrylsäure [Seifen-Öle-Fette-Wachse, **109**, 20 (1983)] alkyliert werden. Von untergeordneter Wichtigkeit ist hingegen die Reaktion von tertiären Aminen mit 3-Chlor-2-hydroxypropansulfonsäure zu amphoteren Tensiden des Sulfobetain-Typs, wie sie z.B. in der britischen Patentanmeldung GB 15 41 427 beschrieben ist.

Alle genannten technischen Verfahren haben den Nachteil, daß die Umsetzung der Stickstoffkomponente mit dem Alkylierungsmittel nicht vollständig verläuft und die Reaktionsprodukte somit zwischen 0.1 und 3 Gew.-% nichtumgesetzte Ausgangsstoffe aufweisen. Da es zur Erzielung geruchlich einwandfreier und toxikologisch unbedenklicher Produkte erforderlich ist, den Gehalt an freien Aminen sowie nicht verbrauchtem Alkylierungsmittel auf ein möglichst niedriges Niveau herabzusenken, hat es in der Vergangenheit nicht an Versuchen gemangelt, dieses Ziel durch Wahl geeigneter Reaktionsbedingungen oder Aufarbeitung der Reaktionsprodukte zu erreichen.

Während die Reduzierung des Gehaltes an freien Aminen in der Lösung amphoterer oder zwitterionischer Tenside z. B. durch Verwendung des Alkylierungsmittels im Überschuß, Reaktionsführung bei pH = 8 bis 10 [DE 29 26 479 A1], destillative Abreicherung oder oxidative Zerstörung des Amins [DE 20 63 422 A1] weitgehend als gelöst angesehen werden kann, führt selbst ein mehrstündiges Erhitzen der Tensidlösungen unter alkalischen Bedingungen nur zu einer geringfügigen Abnahme des Restgehaltes an Alkylierungsmittel.

Das zu lösende technische Problem bestand somit darin, ein Verfahren zu entwickeln, mit dessen Hilfe der Restgehalt an Alkylierungsmittel in Lösungen amphoterer oder zwitterionischer Tenside auf Werte kleiner 0.01 Gew.-% bezogen auf 100 Gew.-% Feststoffgehalt der Tensidlösungen herabgesetzt werden kann.

Gegenstand der Erfindung ist ein Verfahren zur Erniedrigung des Restgehaltes an freiem Alkylierungsmittel in wässrigen Lösungen amphoterer oder zwitterionischer Tenside durch Nachbehandlung mit Ammoniak, einer Aminosäure mit 2 bis 8 Kohlenstoffatomen oder einem Oligopeptid.

Unter amphoteren bzw. zwitterionischen Tensiden sind solche Produkte zu verstehen, die unter alkalischen Bedingungen durch Alkylierung von primären, sekundären oder tertiären Aminen sowie Imidazolinen mit einer Verbindung aus der Gruppe der Halogencarbonsäuren, Halogenhydroxyalkansulfonsäuren und 1,2-ungesättigten Carbonsäuren sowie deren Estern erhältlich sind. Typische Vertreter der stickstoffhaltigen Komponente sind beispielsweise technisches Kokosalkylamin, technisches Kokosfettsäureamidopropyl-dimethylamin oder 1-Hydroxyethyl-2-undecylimidazolin. Unter einem Kokosalkylrest ist dabei ein solcher Rest zu verstehen, der eine C-Kettenverteilung von durchschnittlich 2 Gew.-% $C_{10}$, 57 Gew.-% $C_{12}$, 23 Gew.-% $C_{14}$, 11 Gew.-% $C_{16}$ und 7 Gew.-% $C_{18}$ aufweist.

Typische Beispiele für die zum Einsatz gelangenden Alkylierungsmittel sind 3-Chlor-2-hydroxypropansulfonsäure, Propansulton, Malein- und Crotonsäure sowie deren Ester, insbesondere jedoch Chloressigsäure und Acrylsäure.

Die Nachbehandlung der wässrigen Lösungen amphoterer oder zwitterionischer Tenside kann durch Zusatz von 0.2 bis 10 Gew.-%, vorzugsweise jedoch 0.5 bis 3.0 Gew.-% (bezogen auf die Tensidlösung) Ammoniak, einer Aminosäure mit 2 bis 8 Kohlenstoffatomen oder einem Oligopeptid bevorzugt unter Erwärmen erfolgen.

Ammoniak kommt bei der Nachbehandlung bevorzugt in Form einer 25 bis 55 gew.-%igen wässrigen Lösung zum Einsatz.

Typische Beispiele für Aminosäuren mit 2 bis 8 Kohlenstoffatomen sind Alanin, Arginin, Asparagin, Cystein, Cystin, Dibromtyrosin, Diiodtyrosin, Glutaminsäure, Histidin, Hydroxylysin, Hydroxyprolin, Isoleucin, Leucin, Lysin, Methionin, Phenylalanin, Prolin, Serin, Threonin, Thyroxin, Tryptophan, Tyrosin und Valin. Der Einsatz von Glycin und Glutamin ist dabei bevorzugt.

Geeignet sind auch Oligopeptide, deren Oligomerisierungsgrad niedrig genug ist, um unter Anwendungsbedingungen und in der Anwendungskonzentration vollständig wasserlöslich zu sein. Ferner können auch solche wasserlöslichen Produkte eingesetzt werden, wie sie z. B. bei der partiellen Hydrolyse von Eiweißen, z.B. Gelatine oder Kollagen anfallen [Angew.Chem. **90**, 187, (1978)].

Die Nachbehandlung der wässrigen Lösungen amphoterer oder zwitterionischer Tenside erfolgt, indem man die Tensidlösung mit dem Nachbehandlungsmittel über 0.5 bis 8 h, vorzugsweise 1 bis 4 h bei einem pH-Wert von 7.2 bis 11.5, vorzugsweise 7.5 bis 9.5 auf eine Temperatur von 50 bis 100 °C, vorzugsweise 70 bis 95 °C erwärmt. Allgemein gilt, daß die Senkung des Restgehaltes an Alkylierungsmittel um so rascher erfolgt, je höher die Temperatur bei der Nachbehandlung gehalten wird. Es ist ferner möglich, die Nachbehandlung auch bei einem Druck von 1 bis 2 bar und einer Temperatur bis zu 120 °C im Druckautoklaven durchzuführen.

Durch die Nachbehandlung wird der Restgehalt an freiem Alkylierungsmittel, insbesondere an Chloressigsäure oder Acrylsäure innerhalb von 0.5 bis 8 h auf Werte unterhalb von 0.01 Gew.-% (bezogen auf den Feststoffgehalt) herabgesenkt. Sowohl die zum Einsatz gelangenden Nachbehandlungsmittel, als auch die im Verlauf der Nachbehandlung entstehenden Verbindungen sind toxikologisch unbedenklich und führen weder zu einer anwendungstechnischen, noch zu einer geruchlichen Beeinträchtigung der amphoteren bzw. zwitterionischen Tensidlösungen.

Die nachfolgenden Beispiele sollen den Gegenstand der Erfindung näher erläutern.

## Beispiele

## Synthese amphoterer und zwitterionischer Tenside

**A. Synthese eines Amphotensids vom Alkylbetain-Typ.** 130.4 g (1.1 Mol) Natriumchloracetat wurden in einer mit Thermometer, pH-Elektrode, Tropftrichter und Rückflußkühler versehenen Rührapparatur in 679 g Wasser vorgelegt, mit 235 g (1 Mol) Dimethylkokosalkylamin (C-Kettenverteilung des Kokosalkylrestes : 2 Gew.-% $C_{10}$, 57 Gew.-% $C_{12}$, 23 Gew.-% $C_{14}$, 11 Gew.-% $C_{16}$, 7 Gew.-% $C_{18}$) versetzt und auf 80 °C erwärmt. Nach ca. 2.5 h Reaktionszeit wurde die anfänglich trübe Reaktionsmischung klar. Durch Zusatz 20 gew.-%iger Natriumhydroxidlösung wurde der pH-Wert der Lösung auf 8.0 bis 8.5 gehalten und die Reaktion solange fortgesetzt, bis der Restgehalt an freiem Amin weniger als 0.3 Gew.-% betrug. Nach 8.5 h Reaktionszeit wurde die Umsetzung durch Abkühlen beendet.

| Kenndaten des Produktes: | |
|---|---|
| Feststoff | 34.8 Gew.-% |
| Natriumchlorid | 6.0 Gew.-% |
| Freies Amin | 0.28 Gew.-% = 1.2 mMol/100 g |
| Chloressigsäure | 0.23 Gew.-% entsprechend 0.67 Gew.-% bezogen auf Feststoff |

**B. Synthese eines Amphotensids vom Alkylamidobetain-Typ.** Beispiel 1 wurde unter Verwendung von 204.4 g (1.73 Mol) Natriumchloracetat in 1232 g Wasser und 459.0 g (1.5 Mol) eines Amidierungsproduktes aus 1-Aminopropyl-3-N,N-dimethylamin und gehärteter Kokosfettsäure (C-Kettenverteilung : 7 Gew.-% $C_8$, 6 Gew.-% $C_{10}$, 49 Gew.-% $C_{12}$, 19 Gew.-% $C_{14}$, 9 Gew.-% $C_{16}$, 10 Gew.-% $C_{18}$) bei 90 °C wiederholt. Durch Zusatz von 20 gew.-%iger Natronlauge zu der bei Erreichen der Reaktionstemperatur klaren Lösung wurde der pH-Wert auf 7.5 bis 8 gehalten. Nach 2 h Reaktionszeit wurde die Umsetzung durch Abkühlen beendet.

| Kenndaten des Produktes: | |
|---|---|
| Feststoff<br>Natriumchlorid<br>Freies Amin | 35.3 Gew.-%<br>5.2 Gew.-%<br>< 0.1 Gew.-% |
| Chloressigsäure | 0.84 Gew.-%<br>entsprechend 2.4 Gew.-% bezogen auf Feststoff |

**C. Synthese eines Amphotensids vom Alkylamidobetain-Typ.** Beispiel 2 wurde unter Verwendung von 123.8 g (1.05 Mol) Natriumchloracetat in 750 g Wasser und 306 g (1 Mol) des Amidierungsproduktes wiederholt.

| Kenndaten des Produktes: | |
|---|---|
| Feststoff<br>Natriumchlorid<br>Freies Amin | 34.5 Gew.-%<br>4.8 Gew.-%<br>0.6 Gew.-% |
| Chloressigsäure | 0.39 Gew.-%<br>entsprechend 1.1 Gew.-% bezogen auf Feststoff |

**D. Synthese eines zwitterionischen Tensids vom sogenannten Imidazoliniumbetain-Typ.** 1.155.9 g (0.5 Mol) eines aus Aminoethylethanolamin und gehärteter Kokosfettsäure (C-Kettenverteilung analog Beispiel 2) erhaltenen Imidazolins mit 11.7 Gew.-% Diamid als Nebenprodukt wurden gemäß der DE 30 18 201 A1 mit 6.1 g 50 gew.-%iger Natriumhydroxidlösung und 15.3 g Wasser in einer Apparatur analog Beispiel 1 vorgelegt und 1 h bei 90°C gerührt. Danach wurde eine Lösung von 136.3 g (1.15 Mol) Natriumchloracetat in 423 g Wasser so zugesetzt, daß die Temperatur auf 60°C abfiel und 30 min weitergerührt. Anschließend wurde die Reaktionsmischung mit 46 g 50 gew.-%iger Natriumhydroxidlösung versetzt und die Lösung unter Rühren 3 h bei 60°C gehalten. Nach Erwärmung auf 80°C und Zugabe weiterer 46 g 50 gew.-%iger Natriumhydroxidlösung wurde noch 1.5 h gerührt und die Lösung anschließend abgekühlt.

| Kenndaten des Produktes: | |
|---|---|
| Feststoff<br>Natriumchlorid | 39.5 Gew.-%<br>7.8 Gew.-% |
| Chloressigsäure | 0.57 Gew.-%<br>entsprechend 1.4 Gew.-% bezogen auf Feststoff |

**E. Synthese eines Amphotensids vom sogenannten salzfreien Alkylamidobetain-Typ.** 1.155.9 g (0.5 Mol) eines aus Aminoethylethanolamin und gehärteter Kokosfettsäure (C-Kettenverteilung analog Beispiel 2) erhaltenen Imidazolins mit 11.7 Gew.-% Diamid als Nebenprodukt wurden in 284.4 g Wasser gelöst. Zu der Mischung wurden bei 50 bis 60°C innerhalb von 0.5 h 36.5 g (0.51 Mol) Acrylsäure zugetropft. Anschließend wurde 5 h bei 90°C weitergerührt und danach abgekühlt.

| Kenndaten des Produktes: | |
|---|---|
| Feststoff<br>Acrylsäure | 41.6 Gew.-%<br>1.8 Gew.-% bezogen auf den Feststoff |

Zur Bestimmung des Feststoffgehaltes wurden die Tensidlösungen in einem Trockenschrank bei einer Temperatur von 110°C über 2 h getrocknet und der unter diesen Bedingungen vollständige Wasserverlust durch Differenzwägung ermittelt.

Zur Bestimmung des Gehaltes an freiem Amin wurde die Lösung zunächst mit Salzsäure auf pH = 3.0 eingestellt und das gebildete Aminhydrochlorid mit Natriumhydroxidlösung potentiometrisch titriert.

Die Bestimmung des Gehaltes an Chloressigsäure und Acrylsäure erfolgte ionenchromatographisch über eine Anionaustauschersäule. Die Detektion wurde mit einem Leitfähigkeitsdetektor nach Suppressorreaktion durchgeführt. Die Identifizierung wurde über die Rententionszeiten der Komponenten vorgenommen, die quantitative Auswertung erfolgte im Vergleich zu Eichstandards über die Integration der Peakflächen.

<u>Beispiele 1 - 10</u>

**Nachbehandlung der amphoteren und zwitterionischen Tenside.** Jeweils 200 g der nach A, B. C, D und E erhaltenen Produkte wurden ohne bzw. mit 0.5 bis 1.8 Gew.-% (bezogen auf 100 Gew.-% Tensidlösung) Ammoniak (25 gew.-%ig), einer Aminosäure mit 2 bis 8 Kohlenstoffatomen oder einem Oligopeptid bei 90°C und einem pH-Wert von 8.5 bis 9.0 nachbehandelt. In Abständen von 1 h wurde der Gehalt an Chloressigsäure bzw. Acrylsäure ionenchromatographisch ermittelt. Die Ergebnisse sind in Tab.1 und Tab.2 zusammengefaßt.

Tab.1: Nachbehandlung der amphoteren und zwitterionischen Tenside
Alle Angaben über den Chloressigsäure-Gehalt wurden
auf 100 Gew.-% Feststoffgehalt der Lösungen bezogen.

| Bsp. | Amphotensid gem. | Nachbehandlungs- komponente Gew.% | Chloressigsäuregehalt nach | | | |
| --- | --- | --- | --- | --- | --- | --- |
| | | | 1 h | 2 h | 3 h | 4 h |
| | | | $10^{-4}$ Gew.-% | | | |
| | A | ohne | 5700 | 5400 | 5200 | 4900 |
| 1 | A | 0.5 % Glycin | 3700 | 2400 | 170 | 90 |
| 2 | A | 1.0 % Glycin | 2400 | 900 | 100 | 50 |
| | B | ohne | 20000 | 19000 | 17000 | 14000 |
| 3 | B | 0.5 % Glycin | 2000 | 60 | 60 | 30 |
| 4 | B | 1.0 % Glutamin | 5900 | 2600 | 700 | 100 |
| 5 | B | 1.0 % $NH_3$-Lsg.* | 3700 | 60 | 60 | 30 |
| | C | ohne | 5800 | 4400 | 3800 | 3500 |
| 6 | C | 0.5 % Glycin | 290 | 60 | 30 | 0 |
| | D | ohne | 13000 | 11000 | 10000 | 9000 |
| 7 | D | 1.0 % Glycin | 1260 | 25 | 0 | 0 |
| 8 | D | 1.8 % Glutamin | 3200 | 1500 | 500 | 25 |
| 9 | D | 2.0 % $NH_3$-Lsg.* | 2600 | 130 | 25 | 0 |

\* = $NH_3$-Lösung, 25 gew.-%ig

6

Tab.2: Nachbehandlung der amphoteren und zwitterionischen Tenside
Alle Angaben über den Acrylsäure-Gehalt wurden
auf 100 Gew.-% Feststoffgehalt der Lösungen bezogen.

| Bsp. | Amphotensid gem. | Nachbehandlungs- komponente Gew.% | Acrylsäuregehalt nach | | | |
|------|------|------|------|------|------|------|
| | | | 1 h | 2 h | 3 h | 4 h |
| | | | $10^{-4}$ Gew.-% | | | |
| | E | ohne | 17200 | 17000 | 17000 | 16800 |
| 10 | E | 0.5 % Glycin | 2300 | 950 | 320 | 90 |

**Patentansprüche**

1. Verfahren zur Erniedrigung des Restgehaltes an freiem Alkylierungsmittel in wässrigen Lösungen amphoterer oder zwitterionischer Tenside, dadurch gekennzeichnet, daß man die Lösungen mit Ammoniak, einer Aminosäure mit 2 bis 8 Kohlenstoffatomen oder einem Oligopeptid nachbehandelt.

2. Verfahren nach Anspruch 1, dadurch gekennzeichnet, daß man die Nachbehandlung mit 0.5 bis 3.0 Gew.-% (bezogen auf die Tensidlösung) einer 25 bis 55 gew.%igen Ammoniaklösung, Glycin oder Glutamin durchführt.

3. Verfahren nach einem der Ansprüche 1 und 2, dadurch gekennzeichnet, daß der Restgehalt der Alkylierungsmittel Chloressigsäure und Acrylsäure auf Werte kleiner 0.01 Gew.-% bezogen auf den Feststoffanteil in der Tensidlösung erniedrigt wird.

4. Verfahren nach einem der Ansprüche 1 bis 3, dadurch gekennzeichnet, daß man die Nachbehandlung über 1 bis 4 h bei einem pH-Wert von 7.5 bis 9.5 und einer Temperatur von 70 bis 98°C durchführt.

**Claims**

1. A process for reducing the residual content of free alkylating agent in aqueous solutions of amphoteric or zwitterionic surfactants, characterized in that the solutions are aftertreated with ammonia, an amino acid containing 2 to 8 carbon atoms or an oligopeptide.

2. A process as claimed in claim 1, characterized in that the aftertreatment is carried out with 0.5 to 3.0% by weight (based on the surfactant solution) of a 25 to 55% by weight ammonia solution, glycine or glutamine.

3. A process as claim 1 or 2, characterized in that the residual content of the alkylating agents chloroacetic acid and acrylic acid is reduced to less than 0.01% by weight, based on the solids content of the surfactant solution.

4. A process as claimed in any of claims 1 to 3, characterized in that the aftertreatment is carried out over a period of 1 to 4 h at a pH value of 7.5 to 9.5 and at a temperature of 70 to 98°C.

**Revendications**

1. Procédé pour l'abaissement de la teneur résiduelle en agent d'alcoylation libre dans des solutions aqueuses d'agents tensioactifs amphotères ou zwitterioniques, caractérisé en ce que l'on retraite les solutions avec de l'ammoniac, un aminoacide ayant de 2 à 8 atomes de carbone ou un oligopeptide.

2. Procédé selon la revendication 1, caractérisé en ce que l'on effectue le retraitement avec 0,5 à 3,0 % en poids (rapporté à la solution d'agent tensioactif) d'une solution d'ammoniac à 25-55 % en poids, de glycine ou de glutamine.

3. Procédé selon l'une des revendications 1 et 2, caractérisé en ce que la teneur résiduelle des agents d'alcoylation, l'acide chloracétique et l'acide acrylique, est abaissée à des valeurs inférieures à 0,01 % en poids rapporté au pourcentage de matière solide dans la solution d'agent tensioactif.

4. Procédé selon l'une des revendications 1 à 3, caractérisé en ce que l'on effectue le retraitement pendant 1 à 4 heures à une valeur de pH allant de 7,5 à 9,5 et à une température allant de 70 à 98°C.